(19) Europäisches Patentamt
European Patent Office
Office européen des brevets

(11) **EP 4 711 382 A1**

(12) **EUROPEAN PATENT APPLICATION**
published in accordance with Art. 153(4) EPC

(43) Date of publication:
**18.03.2026 Bulletin 2026/12**

(21) Application number: **24806592.2**

(22) Date of filing: **15.05.2024**

(51) International Patent Classification (IPC):
*C07K 14/47* $^{(2006.01)}$  *C12N 15/12* $^{(2006.01)}$
*C12Q 1/6886* $^{(2018.01)}$  *C12N 15/85* $^{(2006.01)}$
*A61K 38/16* $^{(2006.01)}$  *A61P 35/00* $^{(2006.01)}$
*G01N 33/68* $^{(2006.01)}$  *G01N 33/574* $^{(2006.01)}$

(52) Cooperative Patent Classification (CPC):
**A61K 38/16; A61K 38/17; A61K 48/00;
A61P 35/00; A61P 35/04; C07K 14/435;
C07K 14/47; C12N 15/85; C12Q 1/6886;
G01N 33/575; G01N 33/68**

(86) International application number:
**PCT/CN2024/093260**

(87) International publication number:
**WO 2024/235242 (21.11.2024 Gazette 2024/47)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **15.05.2023  CN 202310544241**

(71) Applicant: **Nanjing Anji Biotechnology Co., Ltd.
Nanjing, Jiangsu 210000 (CN)**

(72) Inventors:
• **XU, Hanmei
  Nanjing, Jiangsu 210000 (CN)**
• **SARRA, Setrerrahmane
  Nanjing, Jiangsu 210000 (CN)**

(74) Representative: **karo IP
Patentanwälte PartG mbB
Steinstraße 16-18
40212 Düsseldorf (DE)**

Remarks:
The complete document including Reference
Table(s) and the Sequence Listing(s) can be
downloaded from the EPO website

(54) **USE OF MICRO PEPTIDE MIAC**

(57)    Use of a micropeptide MIAC, which belongs to the technical field of biomedicine. The present disclosure specifically relates to use of the micropeptide MIAC in preparation of a reagent or a medicament for detecting, preventing or treating tumors. The tumors comprise one or more of solid tumors and hematologic malignancies, such as pancreatic cancer, hepatocellular carcinoma, colorectal cancer, ovarian cancer, cervical cancer, bladder cancer, melanoma, glioblastoma, neuroblastoma, glioma, osteosarcoma, lymphoma, hematologic malignancies, myeloma, cholangiocarcinoma and prostate cancer. The micropeptide MIAC has the effects of inhibiting growth, proliferation and/or migration of various tumor cells, has a wide treatment spectrum, and is suitable for diagnosing, preventing or treating various tumors, specifically malignant tumors.

FIG. 1

**Description**

**TECHNICAL FIELD**

The present application belongs to the technical field of biomedicine, and specifically relates to use of a micropeptide MIAC.

**BACKGROUND**

An open reading frame (ORF) is a potential translatable sequence consisting of a series of in-frame sense codons, and these codons start with an initiation codon and end with a termination codon. Translatable ORFs are typically labeled as coding DNA sequences (CDSs) on an mRNA, and these sequences are responsible for protein translation.

Long noncoding RNAs (lncRNAs) are a type of noncoding RNAs longer than 200 nt, expressed in a tissue- and developmental stage-specific manner, and have been shown to directly regulate multiple functions. lncRNAs also contain short open-reading frames (sORFs), which are generally less than 300 nt in length and were previously thought to lack a coding ability. However, recent studies have shown that some sORFs have the coding ability, and their encoded products are defined as micropeptides.

More and more micropeptides with fewer than 150 amino acids have been discovered in various organisms, from bacteria to humans. The functional diversity of these micropeptides and the emergence of new methods for identifying them have attracted the attention of the scientific community. All these studies indicate that micropeptides translated from sORFs play important regulatory roles in many key physiological processes, such as muscle development, amino acid metabolism, immune regulation, tumor growth, and cell death.

Maolei Zhang et al. identified an lncRNA with a potential coding ability through circular RNA sequencing (circRNAseq) and ribosome nascent chain complex sequencing (RNC-seq) analysis of glioma cells. Experiments revealed that its second exon could form a circular RNA (circRNA), which was translated into a micropeptide of 87 amino acids. In vitro and in vivo experiments demonstrated that this micropeptide could inhibit the proliferative capacity of glioma and interact with a polymerase complex-associated factor (PAF1) to suppress the transcriptional elongation of multiple proto-oncogenes. Clinical sample analysis showed that compared with para-carcinoma tissues, this micropeptide exhibits significantly down-regulated expression in breast cancer, hepatocellular carcinoma, and gastric cancer, suggesting its potential role in the diagnosis, treatment and the like related to malignant tumors (Zhang M, et al. A peptide encoded by circular form of LINC-PINT suppresses oncogenic transcriptional elongation in glioblastoma. Nat Commun. 2018 Oct 26; 9(1):4475.).

In related technologies, for example, the Chinese invention patent with publication number CN112442116A describes use of a micropeptide encoded by an lncRNA in the detection and treatment of malignant tumors such as head and neck cancer, thyroid cancer, and renal carcinoma.

However, different tumor tissues exhibit genetic, phenotypic, and immune heterogeneity, and research on micropeptides, particularly in tumor treatment, remains limited, so that it is necessary to explore their uses in the treatment of different tumors. In order to further expand the spectrum of other tumors where MIAC may play a role, the present disclosure attempts to detect the expression of MIAC in various tumor cells and corresponding normal cells, and systematically evaluates the activity of the micropeptide MIAC in different tumors through in vitro and in vivo experiments, so as to provide a new solution for tumor treatment.

**SUMMARY**

**1. Purpose of the Invention**

The purpose of the present application is to provide use of a micropeptide MIAC. After long-term exploration and continuous attempts, the inventors have found that the micropeptide MIAC having an amino acid sequence as shown in SEQ ID NO: 1 can be used in preparation of a reagent or a medicament for detecting, preventing or treating tumors such as pancreatic cancer, hepatocellular carcinoma, colorectal cancer, ovarian cancer, cervical cancer, bladder cancer, melanoma, glioblastoma, neuroblastoma, glioma, osteosarcoma, lymphoma, hematologic malignancies, myeloma, cholangiocarcinoma and prostate cancer.

**2. Technical Solutions**

To address the above technical problem, the technical solutions adopted in the present application are as follows: The present application provides use of a micropeptide MIAC in preparation of a reagent or a medicament for detecting, preventing or treating tumors, the micropeptide MIAC has an amino acid sequence as shown in SEQ ID NO: 1, and the amino acid sequence of SEQ ID NO: 1 is: MERAGVPGFSPRRSSVEAKMQSTSCSVRKSSTVTAWPAVVLLLSWGQR RGG.

Further, the tumors comprise one or more of pancreatic cancer, hepatocellular carcinoma, colorectal cancer, ovarian cancer, cervical cancer, bladder cancer, melanoma, glioblastoma, neuroblastoma, glioma, osteosarcoma, lymphoma, hematologic malignancies, myeloma, cholangiocarcinoma and prostate cancer.

Further, the reagent or the medicament for preventing or treating tumors comprises at least the micropeptide MIAC having the amino acid sequence as shown in SEQ ID NO: 1 and a pharmaceutically acceptable carrier thereof.

Further, the preventing or treating tumors comprises inhibiting growth, migration and/or proliferation of tumor cells.

Further, the preventing or treating tumors comprises inhibiting the proliferation of tumor cells.

Further, the preventing or treating tumors comprises inhibiting the migration of tumor cells.

Further, the preventing or treating tumors comprises inhibiting the growth of tumor cells, especially the growth of tumor cells in vivo.

Further, the tumor cells in the inhibiting the proliferation of tumor cells include:

cancer cells of the pancreatic cancer, comprising: any one or a combination of MIP-PaCa-2, Aspc-1 and PANC-1;
cancer cells of the hepatocellular carcinoma, comprising: any one or a combination of HepG2, SMMC-7721, HCCLM3, QGY-7701, and Hep3B;
cancer cells of the colorectal cancer, comprising: any one or a combination of LOVO, SW480, and HCT-116;
cancer cells of the ovarian cancer, comprising: SKOV3 and/or A2780;
cancer cells of the cervical cancer, comprising: Hela and/or SIHA;
cancer cells of the bladder cancer, comprising: T24 and/or EJ;
cancer cells of the melanoma, comprising: any one or a combination of A375, SK-mel-2, and M14;
cancer cells of the glioblastoma, comprising: U87-MG;
cancer cells of the neuroblastoma, comprising: SH-SY5Y;
cancer cells of the glioma, comprising: SHG-44;
cancer cells of the osteosarcoma, comprising: any one or a combination of MG-63, Saos-2, and U-2OS;
cancer cells of the lymphoma, comprising: any one or a combination of U937, Raji, U2932, WSU-DLCL2, OCI-LY10, and JeKo-1;
cancer cells of the hematologic malignancies, comprising: any one or a combination of NB4, Reh, K562, Thp-1, JurKat, and HL60;
cancer cells of the myeloma, comprising: any one or a combination of NCI-H929, ARP-1 and RPMI-8226;
cancer cells of the cholangiocarcinoma, comprising: QBC939;
and/or,
cancer cells of the prostate cancer, comprising: Dul45 cells.

Further, the tumor cells in the inhibiting the migration of tumor cells comprise: prostate cancer Du145 cells, cervical cancer Hela cells, osteosarcoma MG-63 cells, ovarian cancer SKOV3 cells, melanoma A375 cells, and/or hepatocellular carcinoma Hep3B cells.

Further, the tumor cells in the inhibiting the growth of tumor cells comprise: hepatocellular carcinoma Hep3B cells.

The present application also provides use of a nucleotide encoding an amino acid sequence as shown in SEQ ID NO: 1 in preparation of a reagent or a medicament for detecting, preventing or treating tumors.

Further, the tumors comprise one or more of pancreatic cancer, hepatocellular carcinoma, colorectal cancer, ovarian cancer, cervical cancer, bladder cancer, melanoma, glioblastoma, neuroblastoma, glioma, osteosarcoma, lymphoma, hematologic malignancies, myeloma, cholangiocarcinoma and prostate cancer.

Further, the nucleotide comprises a nucleotide sequence as shown in SEQ ID NO: 2, and the nucleotide sequence as shown in SEQ ID NO: 2 is: atggagagagcaggggtgcccgggttctctccgcggcgctcatcggtggaggcgaagatgcagagca ccagctgcagtgtcagga agagctccacagtcaccgcctggccagccgtcgtgctgttgctgagctggggacagagaagaggcggatga.

The present application further provides use of a recombinant vector in preparation of a reagent or a medicament for detecting, preventing or treating tumors, and the recombinant vector encodes an amino acid sequence as shown in SEQ ID NO: 1 or comprises a nucleotide sequence as shown in SEQ ID NO: 2.

Further, the tumors comprise one or more of pancreatic cancer, hepatocellular carcinoma, colorectal cancer, ovarian cancer, cervical cancer, bladder cancer, melanoma, glioblastoma, neuroblastoma, glioma, osteosarcoma, lymphoma, hematologic malignancies, myeloma, cholangiocarcinoma and prostate cancer.

A pharmaceutical composition for treating tumors is provided, and the pharmaceutical composition comprises at least the micropeptide MIAC, the nucleotide, or the recombinant vector, and a pharmaceutically acceptable carrier thereof; and the tumors comprise one or more of pancreatic cancer, hepatocellular carcinoma, colorectal cancer, ovarian cancer, cervical cancer, bladder cancer, melanoma, glioblastoma, neuroblastoma, glioma, osteosarcoma, lymphoma, hematologic malignancies, myeloma, cholangiocarcinoma and prostate cancer.

The present application also provides a method for detecting, preventing or treating tumors, and the method comprises administering the micropeptide MIAC having the amino acid sequence as shown in SEQ ID NO: 1 or a pharmaceutical composition compriseing the micropeptide MIAC to a subject with such need.

Further, the subject may be a human, a mouse, or a monkey.

Further, in the method, the micropeptide MIAC is administered to the subject at a dose of 0.1-50 mg/kg, 0.1-20 mg/kg, 0.1-10 mg/kg, 0.1-5 mg/kg, 5-20 mg/kg, 10-20 mg/kg, or 5-15 mg/kg.

**3. Beneficial Effects**

Compared with the related art, the present application has the following beneficial effects.

(1) The expression level of the micropeptide MIAC provided in the present application in human pancreatic cancer, hepatocellular carcinoma, colorectal cancer, ovarian cancer, cervical cancer, bladder cancer, melanoma, glioblastoma, neuroblastoma, glioma, osteosarcoma, lymphoma, hematologic malignancies, myeloma, cholangiocarcinoma and prostate cancer cells is significantly lower than that in the corresponding normal cells.

(2) Regarding the use of micropeptide MIAC provided in the present application, the micropeptide can significantly inhibit the proliferation of tumor cells in pancreatic cancer, hepatocellular carcinoma, colorectal cancer, ovarian cancer, cervical cancer, bladder cancer, melanoma, glioblastoma, neuroblastoma, glioma, osteosarcoma, lymphoma, hematologic malignancies, myeloma, and cholangiocarcinoma, as well as inhibit the migration of tumor cells in prostate cancer, cervical cancer, osteosarcoma, ovarian cancer, melanoma, and hepatocellular carcinoma.

(3) Regarding the use of micropeptide MIAC provided in the present application, the micropeptide can significantly inhibit the in vivo growth of hepatocellular carcinoma, especially tumors derived from hepatocellular carcinoma Hep3B cells.

(4) The micropeptide MIAC provided in the present application has the effects of inhibiting the proliferation and migration of various tumor cells, as well as inhibiting the in vivo tumor growth of hepatocellular carcinoma. It has a wide treatment spectrum, and is suitable for diagnosing, preventing or treating various tumors, specifically malignant tumors.

**BRIEF DESCRIPTION OF THE DRAWINGS**

FIG. 1 shows the expression analysis results of a micropeptide MIAC in different tumor cells and normal cells;

FIG. 2A shows the statistical result of the inhibitory effect of the micropeptide MIAC on the migration activity of prostate cancer Du145 cells;

FIG. 2B shows the cell staining images of a blank group and all administration groups in an experiment of inhibiting the migration activity of prostate cancer Du145 cells by the micropeptide MIAC;

FIG. 3A shows the statistical result of the inhibitory effect of the micropeptide MIAC on the migration activity of cervical cancer Hela cells;

FIG. 3B shows the cell staining images of a blank group and all administration groups in an experiment of inhibiting the migration activity of cervical cancer Hela cells by the micropeptide MIAC;

FIG. 4A shows the statistical result of the inhibitory effect of the micropeptide MIAC on the migration activity of osteosarcoma MG-63 cells;

FIG. 4B shows the cell staining images of a blank group and all administration groups in an experiment of inhibiting the migration activity of osteosarcoma MG-63 cells by the micropeptide MIAC;

FIG. 5A shows the statistical result of the inhibitory effect of the micropeptide MIAC on the migration activity of ovarian cancer SKOV3 cells;

FIG. 5B shows the cell staining images of a blank group and all administration groups in an experiment of inhibiting the migration activity of ovarian cancer SKOV3 cells by the micropeptide MIAC;

FIG. 6A shows the statistical result of the inhibitory effect of the micropeptide MIAC on the migration activity of malignant melanoma A375 cells;

FIG. 6B shows the cell staining images of a blank group and all administration groups in an experiment of inhibiting the migration activity of malignant melanoma A375 cells by the micropeptide MIAC;

FIG. 7A shows the statistical result of the inhibitory effect of the micropeptide MIAC on the migration activity of hepatocellular carcinoma Hep3B cells;

FIG. 7B shows the cell staining images of a blank group and all administration groups in an experiment of inhibiting the migration activity of hepatocellular carcinoma Hep3B cells by the micropeptide MIAC;

FIG. 8 is an in vivo growth curve of a subcutaneously transplanted tumor of hepatocellular carcinoma Hep3B cells in a nude mouse inhibited by the MIAC in Example 4; and

FIG. 9 is a statistical graph of tumor weight on the 22nd day after the MIAC inhibiting the subcutaneously transplanted tumor of hepatocellular carcinoma Hep3B cells in the nude mice in Example 4.

## DETAILED DESCRIPTION

The present application will be further described below in conjunction with specific examples.

It should be noted that terms such as "upper", "lower", "left", "right", and "middle" cited in the specification are only for the sake of clarity in description and are not intended to limit the scope of implementation. Any changes or adjustments to their relative relationships, without substantial changes to the technical content, shall also be deemed to be within the scope of implementation of the present application.

Unless otherwise defined, all technical and scientific terms used herein have the same meaning as that commonly understood by a person skilled in the art to which the present application belongs to; and the term "and/or" as used herein includes any and all combinations of one or more related listed items.

Where specific conditions are not specified in the examples, experiments shall be carried out according to the conventional conditions or the conditions suggested by the manufacturers. For reagents or instruments whose manufacturers are not indicated, they are all conventional products available for purchase in the market.

As used herein, the term "about" is used to provide flexibility and imprecision associated with a given term, metric, or value. Those skilled in the art can easily determine the degree of flexibility for specific variables.

As used herein, the term "at least one of ..." is intended to be synonymous with "one or more of ...". For example, "at least one of A, B and C" explicitly includes only A, only B, only C, and their respective combinations.

Concentrations, amounts, and other numerical data may be presented in a range format herein. It should be understood that such a range format is used merely for convenience and brevity, and should be flexibly interpreted to include not only the numerical values explicitly recited as the limits of the range, but also all individual numerical values or sub-ranges encompassed within the stated range, as if each numerical value and sub-range were explicitly recited. For example, a numerical range of about 1 to about 4.5 should be interpreted to include not only the explicitly recited limits of 1 to about 4.5, but also individual numbers (such as 2, 3, and 4) and sub-ranges (such as 1 to 3, and 2 to 4). The same principle applies to ranges reciting only one numerical value, such as "less than about 4.5", which should be interpreted to include all of the above values and ranges. Moreover, this interpretation should apply regardless of the breadth of the range or feature being described.

A micropeptide inhibiting actin cytoskeleton (MIAC) is a human endogenous protein or polypeptide fragment with 51 amino acids encoded by a long noncoding RNA (lncRNA). The micropeptide MIAC has an amino acid sequence: MERAGVPGFSPRRSSVEAKMQSTSCSVRKSSTVTAWPAVVLLLSWGQRRGG (SEQ ID NO: 1).

The present application provides use of a micropeptide MIAC in preparation of a reagent or a medicament for detecting, preventing or treating tumor diseases.

In some implementations, the tumors comprise solid tumors and hematologic malignancies.

In some implementations, the tumors comprise pancreatic cancer, hepatocellular carcinoma, colorectal cancer, ovarian cancer, cervical cancer, bladder cancer, melanoma, glioblastoma, neuroblastoma, glioma, osteosarcoma, lymphoma, hematologic malignancies, myeloma, cholangiocarcinoma and/or prostate cancer.

The micropeptide MIAC provided in the present application has the effect of inhibiting growth, migration and/or proliferation of tumor cells in vivo and/or in vitro.

In some implementations, the micropeptide MIAC has an inhibitory effect on the proliferation of the following various tumor cells:

cancer cells of pancreatic cancer, comprising: any one or a combination of MIP-PaCa-2, Aspc-1 and PANC-1;

cancer cells of hepatocellular carcinoma, comprising: any one or a combination of HepG2, SMMC-7721, HCCLM3, QGY-7701, and Hep3B;

cancer cells of colorectal cancer, comprising: any one or a combination of LOVO, SW480, and HCT-116;

cancer cells of ovarian cancer, comprising: SKOV3 and/or A2780;

cancer cells of cervical cancer, comprising: Hela and/or SIHA;

cancer cells of bladder cancer, comprising: T24 and/or EJ;

cancer cells of melanoma, comprising: any one or a combination of A375, SK-mel-2, and M14;

cancer cells of glioblastoma, comprising: U87-MG;

cancer cells of neuroblastoma, comprising: SH-SY5Y;

cancer cells of glioma, comprising: SHG-44;

cancer cells of osteosarcoma, comprising: any one or a combination of MG-63, Saos-2, and U-2OS;

cancer cells of lymphoma, comprising: any one or a combination of U937, Raji, U2932, WSU-DLCL2, OCI-LY10, and JeKo-1;
cancer cells of hematologic malignancies, comprising: any one or a combination of NB4, Reh, K562, Thp-1, JurKat, and HL60;
cancer cells of myeloma, comprising: any one or a combination of NCI-H929, ARP-1 and RPMI-8226;
cancer cells of cholangiocarcinoma, comprising: QBC939; and
cancer cells of prostate cancer, comprising: Du145 cells.

The micropeptide MIAC provided in the present application has the effect of inhibiting the proliferation of tumor cells within the range of 50-200 μM, 10-200 μM, or 50-100 μM.

In some implementations, the micropeptide MIAC can also inhibit the migration of tumor cells, specifically comprising: human prostate cancer Dul45 cells, human cervical cancer Hela cells, human osteosarcoma MG-63 cells, human ovarian cancer SKOV3 cells, human malignant melanoma A375 cells, and/or human hepatocellular carcinoma Hep3B cells.

Optionally, the micropeptide MIAC can inhibit the migration of tumor cells within the range of 0.78-50 μM, 0.78-25 μM, 1.56-50 μM, 3.125-50 μM, 6.25-50 μM, 25-50 μM, or 12.5-50 μM.

The micropeptide MIAC provided in the present application can inhibit the in vivo growth of tumor cells. Optionally, the MIAC can inhibit the in vivo tumor growth of human hepatocellular carcinoma cells. In some implementations, the micropeptide MIAC inhibits the in vivo tumor growth of hepatocellular carcinoma Hep3B cells.

Therefore, the present application also provides a method for detecting, preventing or treating tumor diseases, which comprises administering the micropeptide MIAC comprising or having an amino acid sequence as shown in SEQ ID NO: 1 or a pharmaceutical composition comprising the micropeptide MIAC to a subject with such need.

The subject may optionally be a human, a mouse, a monkey, a dog, or a pig. In some implementations, the subject is a human, a monkey, a dog, or a mouse.

When the pharmaceutical composition comprising the micropeptide MIAC is administered to the subject, the pharmaceutical composition further comprises at least one pharmaceutically acceptable excipient.

The term "pharmaceutically acceptable carrier" refers to an excipient or a carrier that does not cause significant irritation to the subject and does not eliminate the biological activity and characteristics of the administered anti-mesothelin antibody or its antigen-binding fragment and/or any additional therapeutic agent in the composition. A pharmaceutically acceptable carrier may enhance or stabilize the composition, or may be used for facilitating the preparation of the composition.

The pharmaceutical composition compriseing the micropeptide MIAC may be administered via an appropriate route based on the specific applicable form, physicochemical properties, and the like of the pharmaceutically acceptable carrier. In some implementations, the pharmaceutical composition may be formulated as a lyophilized preparation or a liquid preparation, which may comprise appropriate formulation additives used in the art. For example, the above pharmaceutical composition typically comprises 1 or more pharmaceutical excipients, for example, sterilized liquids such as water and oils (including oils of petroleum, animal, vegetable, or synthetic origin (e.g., peanut oil, soybean oil, mineral oil, and sesame oil)). In the case of intravenous administration of the above pharmaceutical composition, water is a more representative carrier. In addition, saline solutions as well as aqueous glucose solutions and aqueous glycerol solutions may also be used as liquid carriers, particularly for solutions for injection. Appropriate pharmaceutical excipients are known in the art. If necessary, the composition may also comprise trace amounts of wetting or emulsifying agents, or a pH buffering agent. The pharmaceutical composition is generally administered via a parenteral route, which may be intradermal, intramuscular, intraperitoneal, intravenous, or subcutaneous injection, but is not limited thereto; and for example, it may be administered via infusion or bolus injection, or via an intracranial, intrathecal, or intranasal (e.g., inhalation), intradermal, subcutaneous, or transmucosal route.

When the micropeptide MIAC is used for detecting, preventing, or treating tumors, the dosage varies based on factors such as the specific condition being treated, the severity of the condition, individual patient parameters (including age, physical condition, size, gender, and weight), duration of treatment, nature of concurrent therapy (if any), specific route of administration, and the knowledge of the physician or veterinarian. The dosage is gradually increased until the desired effect is achieved. In some implementations, the dosage of an active ingredient can be determined empirically in individuals who have received one or more micropeptide MIAC administrations.

In some implementations, for example, when 0.1-50 mg/kg, 0.1-20 mg/kg, 0.1-10 mg/kg, 0.1-5 mg/kg, 5-20 mg/kg, 10-20 mg/kg or 5-15 mg/kg of the micropeptide MIAC is administered to a subject, the tumorigenicity of tumor cells can be significantly inhibited, and in particular, the in vivo tumorigenicity of tumor cells can be inhibited.

In some implementations, the dose administered to a subject can be converted between species, such as dose conversion between humans and mice. The conversion method is known in the art. See, for example, *Experimental Methodology of Pharmacology,* Xu Shuyun. Experimental Methodology of Pharmacology (2nd Edition) [M]. People's Medical Publishing House, 1991, the respective contents of which are hereby incorporated by reference.

The technical solutions of the present disclosure will be described more clearly and explicitly below in the following examples in an illustrative manner. It should be understood that these examples are for illustrative purposes only and are by no means intended to limit the scope of protection of the present disclosure. The scope of protection of the present disclosure is limited only by the claims.

**Example 1**

This example detects the expression of a micropeptide MIAC in different tumor cells and normal cells.

(1) Cell culture and RNA extraction

Human pancreatic ductal epithelial cells HPDE and pancreatic cancer cells PANC-1, Aspc-1, human normal hepatocytes L02 and hepatocellular carcinoma cells HepG2, QGY-7701, Hep3B, human normal colon cells CCD-18Co and colorectal cancer cells SW480, HCT-116, human normal ovarian epithelial cells IOSE-80 and ovarian cancer cells SKOV3, A2780, human cervical epithelial cells HCerEpiC and cervical cancer cells Hela, SIHA, human normal bladder epithelial cells SV-HUC-1 and bladder cancer cells T24, EJ, human epidermal keratinocytes HEKa and melanoma cells SK-mel-2, M14, human brain glial cells T98G, glioma cells SHG-44, and neuroblastoma cells SH-SY5Y, human osteoblast cell line hFOB1.19 and osteosarcoma cells MG-63, U-2OS, human T lymphocytes H9 and lymphoma cells U2932, WSU-DLCL2, JeKo-1, human peripheral blood mononuclear cells PBMC and hematologic malignancies cells K562, HL60, NCI-H929, RPMI-8226, human intrahepatic bile duct epithelial cells HIBEpiC and cholangiocarcinoma cells QBC939, as well as human prostate epithelial cells HPEpiC and prostate cancer cells Dul45 were cultured in an incubator at 37°C with 5% $CO_2$ until the density reached 90%, the supernatant was discarded, a certain volume of Trizol (Life Invitrogen) was added, and the total RNA was extracted from different cells according to the instructions.

(2) Primer design

Primers were designed based on the information of nucleotide sequences encoding a micropeptide MIAC, with the sequences as follows:

forward primer (SEQ ID NO. 3): ATGGAGAGAGCAGGGGTGCCCG;
reverse primer (SEQ ID NO. 4): CGCCTCTTCTCTGTCCCCAGCTC.

(3) Real-time quantitative PCR was used for detecting the expression of the micropeptide MIAC in various tumor cells and normal cells.

The total RNA extracted in the above steps was quantified with a NanoDrop ND-1000 nucleic acid quantifier for the purity and concentration of the extracted RNA, and agarose quality control was performed to ensure the integrity of the extracted RNA. The extracted total RNA was reverse-transcribed to synthesize cDNA using a kit with a product name of PrimeScript™ RT reagent Kit with gDNA Eraser (Perfect Real Time) from TaKaRa. A qPCR reaction was carried out using a kit with a product name of SYBR® Premix Ex Taq™ II (Tli RNaseH Plus) from TaKaRa. The reaction system is shown in Table 1. After the above components were mixed evenly, the following procedures were followed: pre-denaturation at 95°C for 30 s, followed by 40 cycles; and 95°C for 5 s, and 60°C for 30 s.

Table 1 PCR reaction system

| Reagent | Usage volume ($\mu$L) |
|---|---|
| SYBR Premix Ex Taq II (Tli RNaseH Plus) (2×) | 12.5 |
| PCR Forward Primer (10 $\mu$M) | 1 |
| PCR Reverse Primer (10 $\mu$M) | 1 |
| DNA template (<100 ng) | 2 |
| Sterilized water | 8.5 |
| Total | 25 |

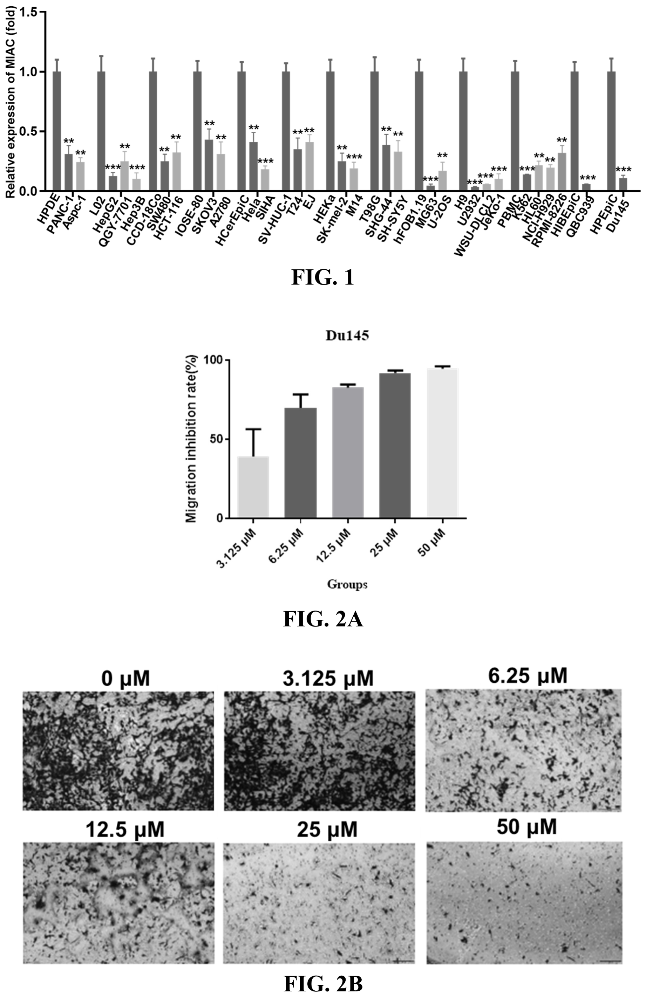

The specificity of the reaction was determined based on a melting curve, and the relative expression of the MIAC was calculated by a formula 2-ΔΔCt. The results are shown in FIG. 1. Compared with the corresponding normal cells, the expression level of the MIAC in human pancreatic cancer, hepatocellular carcinoma, colorectal cancer, ovarian cancer, cervical cancer, bladder cancer, melanoma, glioblastoma, neuroblastoma, glioma, osteosarcoma, myeloma, lymphoma, hematologic malignancies, and cholangiocarcinoma cells was significantly lower than that in normal cells. Therefore, the anti-tumor effect of the MIAC was investigated in the above tumor cells in the future.

**Example 2**

This example provides the inhibitory effect of the micropeptide MIAC on the proliferation of human tumor cells.

Human pancreatic cancer, hepatocellular carcinoma, colorectal cancer, ovarian cancer, cervical cancer, bladder cancer, melanoma, glioblastoma, neuroblastoma, glioma, osteosarcoma, myeloma, lymphoma, hematologic malignancies, and cholangiocarcinoma cells were cultured in an incubator at 37°C with 5% $CO_2$ until the density reached 90%, then digested and collected with trypsin, resuspended in a culture medium, and counted under a microscope; the cell concentration was adjusted to about $1.0 \times 10^5$ cells/mL; and the cell suspension was inoculated into a 96-well plate, with 100 μL per well, and incubated overnight in an incubator at 37°C with 5% $CO_2$. After the cells were completely attached to the wall, 100 μL of micropeptides MIAC with concentrations of 50 μM, 100 μM and 200 μM were added to form an administration group; and a culture medium without any medicament was used as a negative control group. After continuing incubation for 72 h, 100 μL of CCK8 was added to each well of the 96-well plate for dissolving. The absorbance OD was measured at a detection wavelength of 450 nm with a microplate reader, and the proliferation inhibition (PI) was calculated using the formula of PI (%) = 1-administration group/negative control group. The experiment was repeated 3 times independently, the results were expressed as Mean±SD, and a statistical T test was performed, where *P<0.05 indicated a significant difference and **P<0.01 indicated an extremely significant difference. The results are shown in Table 2-Table 52.

Table 2 Inhibitory effect of micropeptide MIAC on the proliferation of human pancreatic cancer cells PANC-1

| Group | | 72 h | |
|---|---|---|---|
| | | OD | Proliferation inhibition (%) |
| Blank well | | 0.291±0.002 | - |
| Negative control group | | 1.048±0.041 | - |
| Administration group | 200 μM | 0.427±0.02** | 59.288±1.909** |
| | 100 μM | 0.708±0.043** | 32.443±4.063** |
| | 50 μM | 1.027±0.073 | 2.036±6.961 |
| Note: Compared with the negative control group, *P<0.05, **P<0.01. | | | |

Table 3 Inhibitory effect of micropeptide MIAC on the proliferation of human pancreatic cancer cells Aspc-1

| Group | | 72 h | |
|---|---|---|---|
| | | OD | Proliferation inhibition (%) |
| Blank well | | 0.291±0.002 | - |
| Negative control group | | 0.948±0.003 | - |
| Administration group | 200 μM | 0.509±0.019** | 46.273±2.035** |
| | 100 μM | 0.711±0.047** | 24.965±4.971** |
| | 50 μM | 0.939±0.015 | 0.949±1.575 |
| Note: Compared with the negative control group, *P<0.05, **P<0.01. | | | |

Table 4 Inhibitory effect of micropeptide MIAC on the proliferation of human pancreatic cancer cells MIP-PaCa-2

| Group | | 72 h | |
|---|---|---|---|
| | | OD | Proliferation inhibition (%) |
| Blank well | | 0.257±0.003 | - |
| Negative control group | | 1.033±0.014 | - |

(continued)

| Group | | 72 h | |
|---|---|---|---|
| | | OD | Proliferation inhibition (%) |
| Administration group | 200 μM | 0.571±0.038** | 44.739±3.716** |
| | 100 μM | 0.866±0.035** | 16.107±3.372** |
| | 50 μM | 0.898±0.035 | 13.041±3.422 |
| Note: Compared with the negative control group, *P<0.05, **P<0.01. | | | |

Table 5 Inhibitory effect of micropeptide MIAC on the proliferation of human esophageal carcinoma cells Eca-109

| Group | | 72 h | |
|---|---|---|---|
| | | OD | Proliferation inhibition (%) |
| Blank well | | 0.291±0.003 | - |
| Negative control group | | 1.064±0.023 | - |
| Administration group | 200 μM | 0.394±0.039** | 63.001±3.636** |
| | 100 μM | 0.656±0.045 ** | 38.315±4.205** |
| | 50 μM | 1.072±0.037 | -0.752±3.493 |
| Note: Compared with the negative control group, *P<0.05, **P<0.01. | | | |

Table 6 Inhibitory effect of micropeptide MIAC on the proliferation of human esophageal carcinoma cells CaES-17

| Group | | 72 h | |
|---|---|---|---|
| | | OD | Proliferation inhibition (%) |
| Blank well | | 0.312±0.003 | - |
| Negative control group | | 1.217±0.030 | - |
| Administration group | 200 μM | 0.466±0.022** | 61.692±1.819** |
| | 100 μM | 0.672±0.051** | 44.770±4.194** |
| | 50 μM | 1.168±0.044 | 4.080±3.650 |
| Note: Compared with the negative control group, *P<0.05, **P<0.01. | | | |

Table 7 Inhibitory effect of micropeptide MIAC on the proliferation of human gastric carcinoma cells BGC-803

| Group | | 72 h | |
|---|---|---|---|
| | | OD | Proliferation inhibition (%) |
| Blank well | | 0.247±0.004 | - |
| Negative control group | | 1.484±0.009 | - |
| Administration group | 200 μM | 0.715±0.015** | 51.853±1.022** |
| | 100 μM | 0.960±0.059** | 35.347±3.963** |
| | 50 μM | 1.379±0.025 | 7.074±1.656 |
| Note: Compared with the negative control group, *P<0.05, **P<0.01. | | | |

Table 8 Inhibitory effect of micropeptide MIAC on the proliferation of human gastric carcinoma cells BGC-823

| Group | | 72 h | |
|---|---|---|---|
| | | OD | Proliferation inhibition (%) |
| Blank well | | 0.291±0.002 | - |
| Negative control group | | 1.128±0.019 | - |
| Administration group | 200 μM | 0.327±0.025** | 71.040±2.217** |
| | 100 μM | 0.556±0.029** | 50.680±2.588** |
| | 50 μM | 0.950±0.045** | 15.780±3.992** |
| Note: Compared with the negative control group, *P<0.05, **P<0.01. | | | |

Table 9 Inhibitory effect of micropeptide MIAC on the proliferation of human hepatocellular carcinoma cells HepG2

| Group | | 72 h | |
|---|---|---|---|
| | | OD | Proliferation inhibition (%) |
| Blank well | | 0.192±0.003 | - |
| Negative control group | | 0.832±0.006 | - |
| Administration group | 200 μM | 0.169±0.015** | 79.696±1.770** |
| | 100 μM | 0.267±0.022** | 67.962±2.687** |
| | 50 μM | 0.575±0.047** | 30.877±5.587** |
| Note: Compared with the negative control group, *P<0.05, **P<0.01. | | | |

Table 10 Inhibitory effect of micropeptide MIAC on the proliferation of human hepatocellular carcinoma cells SMMC-7721

| Group | | 72 h | |
|---|---|---|---|
| | | OD | Proliferation inhibition (%) |
| Blank well | | 0.215±0.002 | - |
| Negative control group | | 1.407±0.004 | - |
| Administration group | 200 μM | 0.674±0.033** | 52.132±2.309** |
| | 100 μM | 1.032±0.011** | 26.670±0.749** |
| | 50 μM | 1.292±0.012 | 8.171±0.860 |
| Note: Compared with the negative control group, *P<0.05, **P<0.01. | | | |

Table 11 Inhibitory effect of micropeptide MIAC on the proliferation of human hepatocellular carcinoma cells HCCLM3

| Group | | 72 h | |
|---|---|---|---|
| | | OD | Proliferation inhibition (%) |
| Blank well | | 0.234±0.002 | - |
| Negative control group | | 1.470±0.025 | - |
| Administration group | 200 μM | 0.706±0.041** | 51.962±2.795** |
| | 100 μM | 0.981±0.019** | 33.273±1.308** |
| | 50 μM | 1.420±0.054 | 3.402±3.691 |
| Note: Compared with the negative control group, *P<0.05, **P<0.01. | | | |

Table 12 Inhibitory effect of micropeptide MIAC on the proliferation of human hepatocellular carcinoma cells QGY-7701

| Group | | 72 h | |
|---|---|---|---|
| | | OD | Proliferation inhibition (%) |
| Blank well | | 0.215±0.002 | - |
| Negative control group | | 1.398±0.009 | - |
| Administration group | 200 μM | 0.520±0.019** | 62.837±1.374** |
| | 100 μM | 0.785±0.030** | 43.862±2.111** |
| | 50 μM | 1.185±0.018** | 15.232±1.260** |
| Note: Compared with the negative control group, *P<0.05, **P<0.01. | | | |

Table 13 Inhibitory effect of micropeptide MIAC on the proliferation of human colon cancer cells LOVO

| Group | | 72 h | |
|---|---|---|---|
| | | OD | Proliferation inhibition (%) |
| Blank well | | 0.247±0.004 | - |
| Negative control group | | 1.339±0.014 | - |
| Administration group | 200 μM | 0.624±0.026** | 53.373±1.965** |
| | 100 μM | 0.970±0.021** | 27.558±1.574** |
| | 50 μM | 1.148±0.008** | 14.289±0.608** |
| Note: Compared with the negative control group, *P<0.05, **P<0.01. | | | |

Table 14 Inhibitory effect of micropeptide MIAC on the proliferation of human colon cancer cells SW480

| Group | | 72 h | |
|---|---|---|---|
| | | OD | Proliferation inhibition (%) |
| Blank well | | 0.192±0.003 | - |
| Negative control group | | 0.812±0.024 | - |
| Administration group | 200 μM | 0.147±0.016** | 81.904±2.015** |
| | 100 μM | 0.283±0.007** | 65.121±0.801** |
| | 50 μM | 0.640±0.032** | 21.215±3.968** |
| Note: Compared with the negative control group, *P<0.05, **P<0.01. | | | |

Table 15 Inhibitory effect of micropeptide MIAC on the proliferation of human colon cancer cells HCT-116

| Group | | 72 h | |
|---|---|---|---|
| | | OD | Proliferation inhibition (%) |
| Blank well | | 0.247±0.004 | - |
| Negative control group | | 1.441±0.013 | - |
| Administration group | 200 μM | 0.688±0.047** | 52.255±3.273** |
| | 100 μM | 0.976±0.038** | 32.269±2.653** |
| | 50 μM | 1.253±0.019** | 13.046±1.340** |
| Note: Compared with the negative control group, *P<0.05, **P<0.01. | | | |

Table 16 Inhibitory effect of micropeptide MIAC on the proliferation of human breast cancer cells MX-1

| Group | | 72 h | |
|---|---|---|---|
| | | OD | Proliferation inhibition (%) |
| Blank well | | 0.257±0.003 | - |
| Negative control group | | 1.069±0.008 | - |
| Administration group | 200 μM | 0.630±0.051** | 41.098±4.761** |
| | 100 μM | 0.808±0.102** | 24.415±9.554** |
| | 50 μM | 1.020±0.028 | 4.584±2.574 |
| Note: Compared with the negative control group, *P<0.05, **P<0.01. | | | |

Table 17 Inhibitory effect of micropeptide MIAC on the proliferation of human breast cancer cells MDA-MB-231

| Group | | 72 h | |
|---|---|---|---|
| | | OD | Proliferation inhibition (%) |
| Blank well | | 0.198±0.003 | - |
| Negative control group | | 1.120±0.009 | - |
| Administration group | 200 μM | 0.206±0.016** | 81.602±1.437** |
| | 100 μM | 0.378±0.058** | 66.270±5.216** |
| | 50 μM | 0.777±0.050** | 30.604±4.508** |
| Note: Compared with the negative control group, *P<0.05, **P<0.01. | | | |

Table 18 Inhibitory effect of micropeptide MIAC on the proliferation of human breast cancer cells MCF-7

| Group | | 72 h | |
|---|---|---|---|
| | | OD | Proliferation inhibition (%) |
| Blank well | | 0.234±0.002 | - |
| Negative control group | | 1.635±0.013 | - |
| Administration group | 200 μM | 0.814±0.047** | 50.194±2.901** |
| | 100 μM | 0.973±0.027** | 40.469±1.643** |
| | 50 μM | 1.281±0.040** | 21.631±2.470** |
| Note: Compared with the negative control group, *P<0.05, **P<0.01. | | | |

Table 19 Inhibitory effect of micropeptide MIAC on the proliferation of human ovarian cancer cells SKOV3

| Group | | 72 h | |
|---|---|---|---|
| | | OD | Proliferation inhibition (%) |
| Blank well | | 0.205±0.002 | - |
| Negative control group | | 1.674±0.017 | - |
| Administration group | 200 μM | 0.727±0.038** | 56.600±2.243** |
| | 100 μM | 0.843±0.049** | 49.652±2.934** |
| | 50 μM | 1.244±0.282** | 25.702±16.864** |
| Note: Compared with the negative control group, *P<0.05, **P<0.01. | | | |

Table 20 Inhibitory effect of micropeptide MIAC on the proliferation of human ovarian cancer cells A2780

| Group | | 72 h | |
|---|---|---|---|
| | | OD | Proliferation inhibition (%) |
| Blank well | | 0.215±0.002 | - |
| Negative control group | | 1.195±0.008 | - |
| Administration group | 200 μM | 0.441±0.018** | 63.114±1.519** |
| | 100 μM | 0.791±0.067** | 33.789±5.608** |
| | 50 μM | 0.985±0.046** | 17.550±3.842** |
| Note: Compared with the negative control group, *P<0.05, **P<0.01. | | | |

Table 21 Inhibitory effect of micropeptide MIAC on the proliferation of human cervical cancer cells Hela

| Group | | 72 h | |
|---|---|---|---|
| | | OD | Proliferation inhibition (%) |
| Blank well | | 0.291±0.002 | - |
| Negative control group | | 1.181±0.018 | - |
| Administration group | 200 μM | 0.532±0.066** | 54.941±5.616** |
| | 100 μM | 0.801±0.039** | 32.185±3.281** |
| | 50 μM | 0.954±0.041** | 19.170±3.432** |
| Note: Compared with the negative control group, *P<0.05, **P<0.01. | | | |

Table 22 Inhibitory effect of micropeptide MIAC on the proliferation of human cervical cancer cells SIHA

| Group | | 72 h | |
|---|---|---|---|
| | | OD | Proliferation inhibition (%) |
| Blank well | | 0.291±0.002 | - |
| Negative control group | | 1.030±0.022 | - |
| Administration group | 200 μM | 0.261±0.052** | 74.693±5.023** |
| | 100 μM | 0.755±0.103** | 26.667±10.000** |
| | 50 μM | 0.844±0.099** | 18.026±9.582** |
| Note: Compared with the negative control group, *P<0.05, **P<0.01. | | | |

Table 23 Inhibitory effect of micropeptide MIAC on the proliferation of human non-small cell lung cancer cells NCI-H1299

| Group | | 72 h | |
|---|---|---|---|
| | | OD | Proliferation inhibition (%) |
| Blank well | | 0.291±0.002 | - |
| Negative control group | | 1.227±0.020 | - |
| Administration group | 200 μM | 0.467±0.012** | 61.950±1.001** |
| | 100 μM | 0.754±0.06** | 38.539±4.892** |
| | 50 μM | 1.090±0.022 | 11.162±1.793 |
| Note: Compared with the negative control group, *P<0.05, **P<0.01. | | | |

Table 24 Inhibitory effect of micropeptide MIAC on the proliferation of human non-small cell lung cancer cells NCI-H1650

| Group | | 72 h | |
|---|---|---|---|
| | | OD | Proliferation inhibition (%) |
| Blank well | | 0.225±0.002 | - |
| Negative control group | | 1.208±0.016 | - |
| Administration group | 200 μM | 0.779±0.057** | 35.559±4.721** |
| | 100 μM | 0.955±0.031** | 20.938±2.565** |
| | 50 μM | 1.127±0.022 | 6.703±1.851 |
| Note: Compared with the negative control group, *P<0.05, **P<0.01. | | | |

Table 25 Inhibitory effect of micropeptide MIAC on the proliferation of human lung giant cell carcinoma cells 95-D

| Group | | 72 h | |
|---|---|---|---|
| | | OD | Proliferation inhibition (%) |
| Blank well | | 0.218±0.003 | - |
| Negative control group | | 1.226±0.010 | - |
| Administration group | 200 μM | 0.341±0.010** | 72.151±0.817** |
| | 100 μM | 0.863±0.054** | 29.589±4.418** |
| | 50 μM | 1.188±0.022 | 3.046±1.818 |
| Note: Compared with the negative control group, *P<0.05, **P<0.01. | | | |

Table 26 Inhibitory effect of micropeptide MIAC on the proliferation of human bladder transitional cell carcinoma cells T24

| Group | | 72 h | |
|---|---|---|---|
| | | OD | Proliferation inhibition (%) |
| Blank well | | 0.312±0.003 | - |
| Negative control group | | 1.134±0.007 | - |
| Administration group | 200 μM | 0.364±0.043** | 67.901±3.786** |
| | 100 μM | 0.730±0.085** | 35.626±7.471** |
| | 50 μM | 1.125±0.010 | 0.794±0.869 |
| Note: Compared with the negative control group, *P<0.05, **P<0.01. | | | |

Table 27 Inhibitory effect of micropeptide MIAC on the proliferation of human bladder cancer cells EJ

| Group | | 72 h | |
|---|---|---|---|
| | | OD | Proliferation inhibition (%) |
| Blank well | | 0.312±0.003 | - |
| Negative control group | | 1.123±0.007 | - |
| Administration group | 200 μM | 0.628±0.045** | 44.125±3.964** |
| | 100 μM | 0.891±0.009** | 20.682±0.761** |
| | 50 μM | 1.126±0.017 | -0.208±1.490 |
| Note: Compared with the negative control group, *P<0.05, **P<0.01. | | | |

Table 28 Inhibitory effect of micropeptide MIAC on the proliferation of human malignant melanoma cells A375

| Group | | 72 h | |
|---|---|---|---|
| | | OD | Proliferation inhibition (%) |
| Blank well | | 0.304±0.003 | - |
| Negative control group | | 1.544±0.027 | - |
| Administration group | 200 μM | 0.911±0.071** | 41.032±4.600** |
| | 100 μM | 1.194±0.043** | 22.664±2.800** |
| | 50 μM | 1.310±0.069** | 15.174±4.488** |
| Note: Compared with the negative control group, *P<0.05, **P<0.01. | | | |

Table 29 Inhibitory effect of micropeptide MIAC on the proliferation of human malignant melanoma cells SK-mel-2

| Group | | 72 h | |
|---|---|---|---|
| | | OD | Proliferation inhibition (%) |
| Blank well | | 0.234±0.002 | - |
| Negative control group | | 1.567±0.015 | - |
| Administration group | 200 μM | 0.570±0.011** | 63.632±0.672** |
| | 100 μM | 0.822±0.060** | 47.554±3.816** |
| | 50 μM | 1.302±0.043** | 16.908±2.713** |
| Note: Compared with the negative control group, *P<0.05, **P<0.01. | | | |

Table 30 Inhibitory effect of micropeptide MIAC on the proliferation of human malignant melanoma cells M14

| Group | | 72 h | |
|---|---|---|---|
| | | OD | Proliferation inhibition (%) |
| Blank well | | 0.234±0.002 | - |
| Negative control group | | 1.690±0.029 | - |
| Administration group | 200 μM | 0.639±0.046** | 62.162±2.695** |
| | 100 μM | 1.046±0.060** | 38.075±3.523** |
| | 50 μM | 1.585±0.030 | 6.195±1.791 |
| Note: Compared with the negative control group, *P<0.05, **P<0.01. | | | |

Table 31 Inhibitory effect of micropeptide MIAC on the proliferation of human cerebral astrocytic glioblastoma cells U87-MG

| Group | | 72 h | |
|---|---|---|---|
| | | OD | Proliferation inhibition (%) |
| Blank well | | 0.225±0.002 | - |
| Negative control group | | 1.040±0.038 | - |
| Administration group | 200 μM | 0.656±0.030** | 36.955±2.912** |
| | 100 μM | 0.785±0.029** | 24.519±2.802** |
| | 50 μM | 0.951±0.019 | 8.590±1.860 |
| Note: Compared with the negative control group, *P<0.05, **P<0.01. | | | |

Table 32 Inhibitory effect of micropeptide MIAC on the proliferation of human neuroblastoma cells SH-SY5Y

| Group | | 72 h | |
|---|---|---|---|
| | | OD | Proliferation inhibition (%) |
| Blank well | | 0.225±0.002 | - |
| Negative control group | | 1.170±0.009 | - |
| Administration group | 200 μM | 0.665±0.011** | 43.175±0.922** |
| | 100 μM | 0.822±0.108** | 29.752±9.195** |
| | 50 μM | 1.033±0.022 | 11.656±1.912 |
| Note: Compared with the negative control group, *P<0.05, **P<0.01. | | | |

Table 33 Inhibitory effect of micropeptide MIAC on the proliferation of human glioma cells SHG-44

| Group | | 72 h | |
|---|---|---|---|
| | | OD | Proliferation inhibition (%) |
| Blank well | | 0.218±0.003 | - |
| Negative control group | | 1.276±0.014 | - |
| Administration group | 200 μM | 0.393±0.021** | 69.183±1.606** |
| | 100 μM | 0.838±0.067** | 34.369±5.261** |
| | 50 μM | 1.201±0.026 | 5.876±2.067 |
| Note: Compared with the negative control group, *P<0.05, **P<0.01. | | | |

Table 34 Inhibitory effect of micropeptide MIAC on the proliferation of human osteosarcoma cells MG-63

| Group | | 72 h | |
|---|---|---|---|
| | | OD | Proliferation inhibition (%) |
| Blank well | | 0.198±0.003 | - |
| Negative control group | | 1.279±0.025 | - |
| Administration group | 200 μM | 0.365±0.009** | 71.488±0.665** |
| | 100 μM | 0.691±0.033** | 45.999±2.541** |
| | 50 μM | 1.049±0.011** | 17.983±0.824** |
| Note: Compared with the negative control group, *P<0.05, **P<0.01. | | | |

Table 35 Inhibitory effect of micropeptide MIAC on the proliferation of human osteogenic sarcoma cells Saos-2

| Group | | 72 h | |
|---|---|---|---|
| | | OD | Proliferation inhibition (%) |
| Blank well | | 0.312±0.003 | - |
| Negative control group | | 1.270±0.023 | - |
| Administration group | 200 μM | 0.627±0.052** | 50.669±4.107** |
| | 100 μM | 0.946±0.016** | 25.558±1.220** |
| | 50 μM | 1.198±0.067 | 5.694±5.302 |
| Note: Compared with the negative control group, *P<0.05, **P<0.01. | | | |

Table 36 Inhibitory effect of micropeptide MIAC on the proliferation of human osteosarcoma cells U-2OS

| Group | | 72 h | |
|---|---|---|---|
| | | OD | Proliferation inhibition (%) |
| Blank well | | 0.323±0.002 | - |
| Negative control group | | 0.922±0.007 | - |
| Administration group | 200 μM | 0.350±0.013** | 61.989±1.457** |
| | 100 μM | 0.596±0.061** | 35.298±6.605** |
| | 50 μM | 0.887±0.024 | 3.761±2.606 |
| Note: Compared with the negative control group, *P<0.05, **P<0.01. | | | |

Table 37 Inhibitory effect of micropeptide MIAC on the proliferation of human histiocytic lymphoma cells U937

| Group | | 72 h | |
|---|---|---|---|
| | | OD | Proliferation inhibition (%) |
| Blank well | | 0.257±0.003 | - |
| Negative control group | | 1.198±0.025 | - |
| Administration group | 200 μM | 0.684±0.046** | 42.877±3.800** |
| | 100 μM | 0.821±0.047** | 31.469±3.907** |
| | 50 μM | 1.069±0.028 | 10.740±2.338 |
| Note: Compared with the negative control group, *P<0.05, **P<0.01. | | | |

Table 38 Inhibitory effect of micropeptide MIAC on the proliferation of human lymphoma cells Raji

| Group | | 72 h | |
|---|---|---|---|
| | | OD | Proliferation inhibition (%) |
| Blank well | | 0.323±0.002 | - |
| Negative control group | | 0.989±0.016 | - |
| Administration group | 200 μM | 0.421±0.029** | 57.432±2.890** |
| | 100 μM | 0.687±0.019** | 30.502±1.951** |
| | 50 μM | 0.964±0.037 | 2.494±3.724 |
| Note: Compared with the negative control group, *P<0.05, **P<0.01. | | | |

Table 39 Inhibitory effect of micropeptide MIAC on the proliferation of human diffuse large B-cell lymphoma cells U2932

| Group | | 72 h | |
|---|---|---|---|
| | | OD | Proliferation inhibition (%) |
| Blank well | | 0.183±0.002 | - |
| Negative control group | | 0.833±0.025 | - |
| Administration group | 200 μM | 0.167±0.025 ** | 79.952±3.030** |
| | 100 μM | 0.275±0.038** | 67.027±4.613** |
| | 50 μM | 0.426±0.019** | 48.860±2.290** |
| Note: Compared with the negative control group, *P<0.05, **P<0.01. | | | |

Table 40 Inhibitory effect of micropeptide MIAC on the proliferation of human diffuse large B-cell lymphoma cells WSU-DLCL2

| Group | | 72 h | |
|---|---|---|---|
| | | OD | Proliferation inhibition (%) |
| Blank well | | 0.310±0.007 | - |
| Negative control group | | 0.989±0.005 | - |
| Administration group | 200 μM | 0.264±0.056* * | 73.306±5.703** |
| | 100 μM | 0.684±0.039** | 30.839±3.947** |
| | 50 μM | 0.908±0.061 | 8.156±6.184 |
| Note: Compared with the negative control group, *P<0.05, **P<0.01. | | | |

Table 41 Inhibitory effect of micropeptide MIAC on the proliferation of human diffuse large B-cell lymphoma cells OCI-LY10

| Group | | 72 h | |
|---|---|---|---|
| | | OD | Proliferation inhibition (%) |
| Blank well | | 0.192±0.003 | - |
| Negative control group | | 0.826±0.030 | - |
| Administration group | 200 μM | 0.376±0.019** | 54.421±2.289** |
| | 100 μM | 0.598±0.033** | 27.574±4.002** |
| | 50 μM | 0.719±0.028** | 12.959±3.388** |
| Note: Compared with the negative control group, *P<0.05, **P<0.01. | | | |

Table 42 Inhibitory effect of micropeptide MIAC on the proliferation of human mantle cell lymphoma cells JeKo-1

| Group | | 72 h | |
|---|---|---|---|
| | | OD | Proliferation inhibition (%) |
| Blank well | | 0.310±0.007 | - |
| Negative control group | | 1.273±0.041 | - |
| Administration group | 200 μM | 0.496±0.052** | 61.021±4.051** |
| | 100 μM | 0.820±0.078** | 35.602±6.132** |
| | 50 μM | 1.174±0.027 | 7.827±2.109 |
| Note: Compared with the negative control group, *P<0.05, **P<0.01. | | | |

Table 43 Inhibitory effect of micropeptide MIAC on the proliferation of human acute promyelocytic leukemia cells NB4

| Group | | 72 h | |
|---|---|---|---|
| | | OD | Proliferation inhibition (%) |
| Blank well | | 0.257±0.003 | - |
| Negative control group | | 1.364±0.016 | - |
| Administration group | 200 μM | 0.624±0.052** | 54.217±3.817** |
| | 100 μM | 0.826±0.066** | 39.404±4.803** |
| | 50 μM | 1.177±0.042** | 13.689±3.103** |
| Note: Compared with the negative control group, *P<0.05, **P<0.01. | | | |

Table 44 Inhibitory effect of micropeptide MIAC on the proliferation of human acute non-T non-B lymphocytic leukemia cells Reh

| Group | | 72 h | |
|---|---|---|---|
| | | OD | Proliferation inhibition (%) |
| Blank well | | 0.323±0.002 | - |
| Negative control group | | 0.989±0.016 | - |
| Administration group | 200 μM | 0.421±0.029** | 57.432±2.890** |
| | 100 μM | 0.687±0.019** | 30.502±1.951** |
| | 50 μM | 0.964±0.037 | 2.494±3.724 |
| Note: Compared with the negative control group, *P<0.05, **P<0.01. | | | |

Table 45 Inhibitory effect of micropeptide MIAC on the proliferation of human chronic myeloid leukemia cells K562

| Group | | 72 h | |
|---|---|---|---|
| | | OD | Proliferation inhibition (%) |
| Blank well | | 0.183±0.002 | - |
| Negative control group | | 1.206±0.004 | - |
| Administration group | 200 μM | 0.338±0.025** | 71.993±2.074** |
| | 100 μM | 0.544±0.062** | 54.907±5.168** |
| | 50 μM | 1.004±0.012** | 16.699±0.990** |
| Note: Compared with the negative control group, *P<0.05, **P<0.01. | | | |

Table 46 Inhibitory effect of micropeptide MIAC on the proliferation of human monocytic leukemia cells Thp-1

| Group | | 72 h | |
|---|---|---|---|
| | | OD | Proliferation inhibition (%) |
| Blank well | | 0.310±0.007 | - |
| Negative control group | | 1.335±0.012 | - |
| Administration group | 200 μM | 0.681±0.045** | 49.014±3.359** |
| | 100 μM | 0.841±0.058** | 36.979±4.341** |
| | 50 μM | 1.265±0.059 | 5.243±4.414 |
| Note: Compared with the negative control group, *P<0.05, **P<0.01. | | | |

Table 47 Inhibitory effect of micropeptide MIAC on the proliferation of human T-cell acute lymphoblastic leukemia cells JurKat

| Group | | 72 h | |
|---|---|---|---|
| | | OD | Proliferation inhibition (%) |
| Blank well | | 0.291±0.002 | - |
| Negative control group | | 1.079±0.029 | - |
| Administration group | 200 μM | 0.559±0.038** | 48.146±3.543** |
| | 100 μM | 0.730±0.032** | 32.355±2.996** |
| | 50 μM | 0.984±0.032 | 8.776±3.008 |
| Note: Compared with the negative control group, *P<0.05, **P<0.01. | | | |

Table 48 Inhibitory effect of micropeptide MIAC on the proliferation of human myeloid leukemia cells HL60

| Group | | 72 h | |
|---|---|---|---|
| | | OD | Proliferation inhibition (%) |
| Blank well | | 0.215±0.002 | - |
| Negative control group | | 1.691±0.018 | - |
| Administration group | 200 μM | 0.549±0.023** | 67.560±1.336** |
| | 100 μM | 0.725±0.027** | 57.115±1.600** |
| | 50 μM | 1.378±0.029** | 18.506±1.732** |
| Note: Compared with the negative control group, *P<0.05, **P<0.01. | | | |

Table 49 Inhibitory effect of micropeptide MIAC on the proliferation of human myeloma cells NCI-H929

| Group | | 72 h | |
|---|---|---|---|
| | | OD | Proliferation inhibition (%) |
| Blank well | | 0.183±0.002 | - |
| Negative control group | | 1.089±0.005 | - |
| Administration group | 200 μM | 0.263±0.026** | 75.849±2.402** |
| | 100 μM | 0.427±0.016** | 60.759±1.429** |
| | 50 μM | 0.860±0.012** | 21.028±1.128** |
| Note: Compared with the negative control group, *P<0.05, **P<0.01. | | | |

Table 50 Inhibitory effect of micropeptide MIAC on the proliferation of human myeloma cells ARP-1

| Group | | 72 h | |
|---|---|---|---|
| | | OD | Proliferation inhibition (%) |
| Blank well | | 0.310±0.007 | - |
| Negative control group | | 1.081±0.013 | - |
| Administration group | 200 μM | 0.433±0.013** | 59.914±1.171** |
| | 100 μM | 0.692±0.047** | 36.016±4.355** |
| | 50 μM | 0.985±0.008 | 8.850±0.719 |
| Note: Compared with the negative control group, *P<0.05, **P<0.01. | | | |

Table 51 Inhibitory effect of micropeptide MIAC on the proliferation of peripheral blood B lymphocytes RPMI-8226 in human multiple myeloma

| Group | | 72 h | |
|---|---|---|---|
| | | OD | Proliferation inhibition (%) |
| Blank well | | 0.205±0.002 | - |
| Negative control group | | 2.054±0.033 | - |
| Administration group | 200 μM | 0.522±0.028** | 74.586±1.366** |
| | 100 μM | 1.001±0.027** | 51.282±1.326** |
| | 50 μM | 2.008±0.023 | 2.256±1.100 |
| Note: Compared with the negative control group, *P<0.05, **P<0.01. | | | |

Table 52 Inhibitory effect of micropeptide MIAC on the proliferation of human cholangiocarcinoma cells QBC939

| Group | | 72 h | |
|---|---|---|---|
| | | OD | Proliferation inhibition (%) |
| Blank well | | 0.218±0.003 | - |
| Negative control group | | 1.134±0.029 | - |
| Administration group | 200 μM | 0.468±0.045** | 58.718±3.932** |
| | 100 μM | 0.757±0.028** | 33.255±2.467** |
| | 50 μM | 1.13±0.053 | 0.294±4.643 |
| Note: Compared with the negative control group, *P<0.05, **P<0.01. | | | |

As shown in Tables 2-52, compared with the negative control group, the micropeptide MIAC at doses of 50-200 μM can significantly inhibit the proliferation of human pancreatic cancer, hepatocellular carcinoma, colorectal cancer, ovarian cancer, cervical cancer, bladder cancer, melanoma, glioma, neuroblastoma, osteosarcoma, myeloma, lymphoma, leukemia, and cholangiocarcinoma cells to varying degrees, demonstrating a dose-dependent relationship.

**Example 3**

This example provides the inhibitory effect of the micropeptide MIAC on the migration of human tumor cells.
Human prostate cancer, cervical cancer, osteosarcoma, ovarian cancer, melanoma, and hepatocellular carcinoma cells were inoculated into transwell chambers, with 100 μL per well; for an administration group, 100 μL of a micropeptide MIAC was added to each of the chambers at a different dose; and no medicament was added to a blank group. Then, 0.6 mL of a complete medium compriseing 10% FBS was added to transwell lower chambers to stimulate cell migration, followed by incubation at 37°C with 5% $CO_2$ for 48 h. The culture medium in the wells was discarded, and the cells were fixed with methanol at room temperature for 30 min, stained with 0.1% crystal violet at room temperature for 10 min, and rinsed thoroughly with clear water; the unmigrated cells in the upper layer were wiped off with a cotton swab; and the migrated cells were observed under a microscope, and four visual fields were selected for photography and counting. The migration inhibition rate (MI) was calculated using the following formula:

$$MI(\%) = \left(1 - \frac{N_{test}}{N_{control}}\right) \times 100\%$$

where $N_{test}$ represents the number of migrated cells in the administration group, and $N_{control}$ represents the number of migrated cells in the blank control group.
The results are shown in Tables 53-58 and FIG. 2-FIG. 7.

Table 53 Inhibitory effect of MIAC on the migration activity of prostate cancer Du145 cells

| Group | Medicament | Dose (μmol/Lμmol/L) | Number of migrated cells | Inhibition rate (%) |
|---|---|---|---|---|
| G1 | None | 0 | 574.8±106.9 | - |
| G2 | | 3.125 | 349.3±98.5 | 39.23 |
| G3 | | 6.25 | 173.8±48.8 | 69.77 |
| G4 | MIAC | 12.5 | 100.5±11.8 | 82.51 |
| G5 | | 25 | 46.8±8.5 | 91.87 |
| G6 | | 50 | 31.3±8.4 | 94.56 |

FIG. 2A corresponds to the statistical graph of all the groups in Table 53; and FIG. 2B corresponds to the staining images of cells in all the groups in Table 53 above.

Table 54 Inhibitory effect of MIAC on the migration activity of cervical cancer Hela cells

| Group | Medicament | Dose (μmol/L) | Number of migrated cells | Inhibition rate (%) |
|---|---|---|---|---|
| GI | None | 0 | 544.5±132.8 | - |

(continued)

| Group | Medicament | Dose ($\mu$mol/L) | Number of migrated cells | Inhibition rate (%) |
|---|---|---|---|---|
| G2 | | 6.25 | 475.0±53.3 | 12.76 |
| G3 | MIAC | 12.5 | 296.8±45.0 | 39.23 |
| G4 | | 25 | 360.3±119.4 | 33.83 |
| G5 | | 50 | 210.5±66.6 | 61.30 |

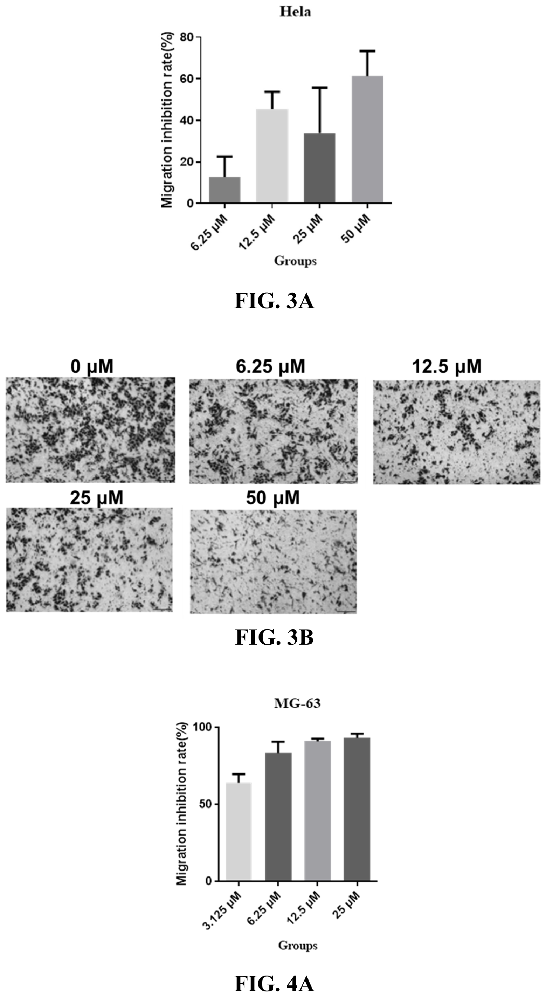

FIG. 3A corresponds to the statistical graph of all the groups in Table 54; and FIG. 3B corresponds to the staining images of cells in all the groups in Table 54 above.

Table 55 Inhibitory effect of MIAC on the migration activity of osteosarcoma MG-63 cells

| Group | Medicament | Dose ($\mu$mol/L) | Number of migrated cells | Inhibition rate (%) |
|---|---|---|---|---|
| GI | None | 0 | 360.3±123.0 | - |
| G2 | | 3.125 | 129.5±19.9 | 64.05 |
| G3 | MIAC | 6.25 | 60.5±26.7 | 83.21 |
| G4 | | 12.5 | 32.5±6.0 | 90.98 |
| G5 | | 25 | 24.8±9.6 | 93.13 |

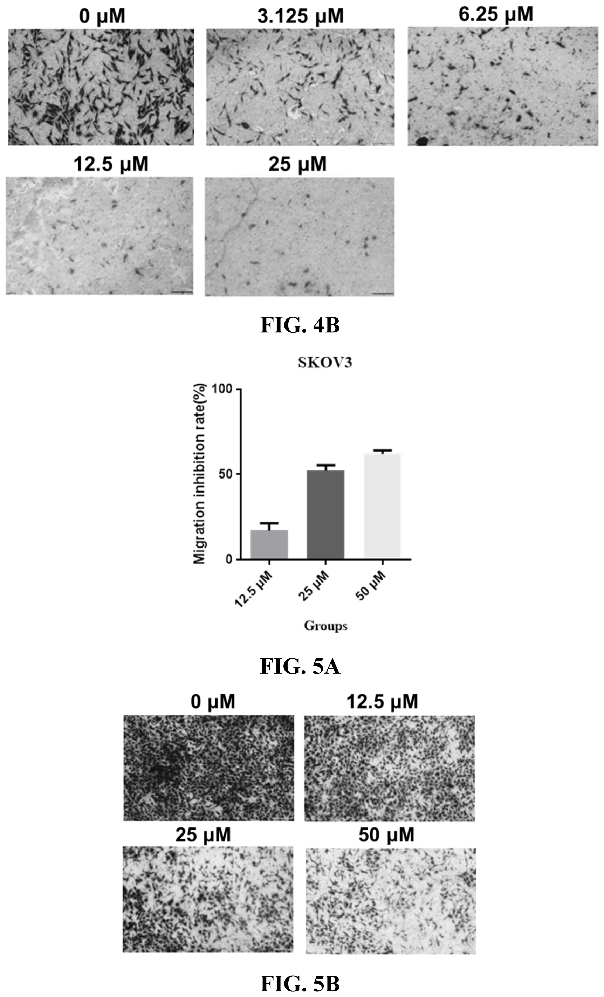

FIG. 4A corresponds to the statistical graph of all the groups in Table 55; and FIG. 4B corresponds to the staining images of cells in all the groups in Table 55 above.

Table 56 Inhibitory effect of MIAC on the migration activity of ovarian cancer SKOV3 cells

| Group | Medicament | Dose ($\mu$mol/L) | Number of migrated cells | Inhibition rate (%) |
|---|---|---|---|---|
| G1 | None | 0 | 1095.3±63.9 | - |
| G2 | | 12.5 | 907.3±46.5 | 17.17 |
| G3 | MIAC | 25 | 523.8±35.3 | 52.28 |
| G4 | | 50 | 415.8±21.7 | 62.04 |

FIG. 5A corresponds to the statistical graph of all the groups in Table 56; and FIG. 5B corresponds to the staining images of cells in all the groups in Table 56 above.

Table 57 Inhibitory effect of MIAC on the migration activity of malignant melanoma A375 cells

| Group | Medicament | Dose ($\mu$mol/L) | Number of migrated cells | Inhibition rate (%) |
|---|---|---|---|---|
| G1 | None | 0 | 258.8±26.3 | - |
| G2 | | 12.5 | 139.3±28.5 | 46.18 |
| G3 | MIAC | 25 | 164.3±40.1 | 36.52 |
| G4 | | 50 | 132.8±35.1 | 48.70 |

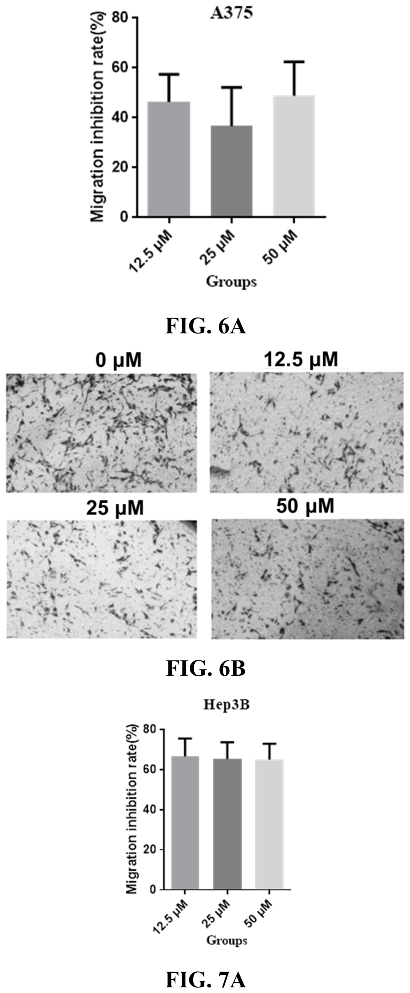

FIG. 6A corresponds to the statistical graph of all the groups in Table 57; and FIG. 6B corresponds to the staining images of cells in all the groups in Table 57 above.

Table 58 Inhibitory effect of MIAC on the migration activity of hepatocellular carcinoma Hep3B cells

| Group | Medicament | Dose ($\mu$mol/L) | Number of migrated cells | Inhibition rate (%) |
|---|---|---|---|---|
| G1 | None | 0 | 331.0±18.2 | - |
| G2 | | 12.5 | 110.8±29.3 | 66.54 |
| G3 | MIAC | 25 | 114.8±27.1 | 65.33 |
| G4 | | 50 | 116.0±26.1 | 64.95 |

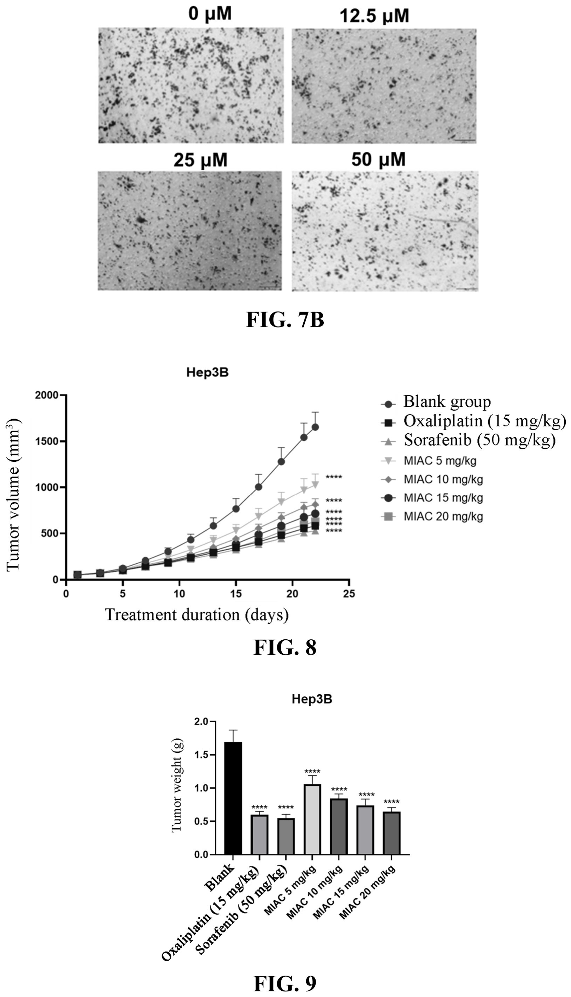

FIG. 7A corresponds to the statistical graph of all the groups in Table 58; and FIG. 7B corresponds to the staining images of cells in all the groups in Table 58 above.

It can be seen from Tables 53-58 and FIG. 2-FIG. 7 that, compared with the blank control group, the micropeptide MIAC has a significant inhibitory effect on the migration of human prostate cancer Du145 cells, human osteosarcoma MG-63 cells, human ovarian cancer SKOV3 cells, human hepatocellular carcinoma Hep3B cells, human melanoma A375 cells, and human cervical cancer Hela cells within a certain range, and the inhibition rate is dose-dependent, indicating that the micropeptide MIAC can be developed into anti-tumor medicaments by inhibiting the migration of the above malignant tumor cells.

**Example 4**

This example provides the inhibitory effect of a micropeptide MIAC on the in vivo growth of human tumor cells.

Human hepatocellular carcinoma Hep3B cells were cultured in large quantities and digested with a 0.25% trypsin solution; and the cell suspension after digestion termination was centrifuged at 1000 rpm for 5 minutes, and the cells were resuspended in a serum-free MEM medium and then counted to adjust the cell concentration to $5 \times 10^7$ cells/mL. Each BALB/c nude mouse (4-6 weeks old, female, weighing 14-16 g, adaptively housed in an SPF animal breeding room for 1 week) was inoculated with 100 μL of the cell suspension in the left axilla, with an inoculated cell amount of $5 \times 10^6$ cells. After inoculation, the tumor growth at the inoculation site of nude mice was closely observed. On the 7th day after inoculation, white nodules appeared at the inoculation site, which could be moved subcutaneously upon touch. As the tumor tissue grew, a hard tumor mass gradually formed at the inoculation site. Approximately 14 days later, the average volume of the tumor tissue reached 100 mm$^3$, the BALB/c nude mice were randomly divided into 7 groups (a normal saline group was blank control, the dosage of the micropeptide MIAC at 5 mg/kg was considered as a low dose, the dosage of the micropeptide MIAC at 10 mg/kg was considered as a medium dose, the dosage of the micropeptide MIAC at 15 mg/kg was considered as a medium-high dose, the dosage of the micropeptide MIAC at 20 mg/kg was considered as a high dose, and 15 mg/kg oxaliplatin and 50 mg/kg sorafenib were positive controls), and there were 10 mice in the blank group and 6 mice in each of the other groups, with the body weight of the mice being about 20 g at the start of administration. The volume of the transplanted tumor was measured and recorded every two days, and the formula for calculating the tumor volume (TV) was: tumor volume=$0.5 \times a \times b^2$, where a is the length (mm) of the transplanted tumor and b is the width (mm) of the transplanted tumor.

The results are shown in FIG. 8 and FIG. 9. Compared with the blank control group, the micropeptide MIAC can significantly inhibit the in vivo tumor growth of human hepatocellular carcinoma Hep3B cells to varying degrees, demonstrating a dose-dependent relationship. At a dose of 20 mg/kg, its therapeutic effect on tumors is similar to that of positive medicaments, indicating that the micropeptide MIAC has a good anti-tumor effect in vivo.

**Claims**

1. Use of a micropeptide MIAC in preparation of a reagent or a medicament for detecting, preventing or treating tumors, wherein the micropeptide MIAC has an amino acid sequence as shown in SEQ ID NO: 1; and the tumors comprise one or more of pancreatic cancer, hepatocellular carcinoma, colorectal cancer, ovarian cancer, cervical cancer, bladder cancer, melanoma, glioblastoma, neuroblastoma, glioma, osteosarcoma, lymphoma, hematologic malignancies, myeloma, cholangiocarcinoma and prostate cancer.

2. The use of a micropeptide MIAC in preparation of a reagent or a medicament for detecting, preventing or treating tumors according to claim 1, wherein the reagent or the medicament for preventing or treating tumors comprises at least the micropeptide MIAC and a pharmaceutically acceptable carrier thereof.

3. The use of a micropeptide MIAC in preparation of a reagent or a medicament for detecting, preventing or treating tumors according to claim 1 or 2, wherein the preventing or treating tumors comprises inhibiting growth, migration and/or proliferation of the tumor cells.

4. The use of a micropeptide MIAC in preparation of a reagent or a medicament for detecting, preventing or treating tumors according to claim 3, wherein the preventing or treating tumors comprises inhibiting the proliferation of the tumor cells,

cancer cells of the pancreatic cancer comprise any one or a combination of MIP-PaCa-2, Aspc-1 and PANC-1;
cancer cells of the hepatocellular carcinoma comprise any one or a combination of HepG2, SMMC-7721, HCCLM3, QGY-7701, and Hep3B;
cancer cells of the colorectal cancer comprise any one or a combination of LOVO, SW480, and HCT-116;

cancer cells of the ovarian cancer comprise SKOV3 and/or A2780;

cancer cells of the cervical cancer comprise Hela and/or SIHA;

cancer cells of the bladder cancer comprise T24 and/or EJ;

cancer cells of the melanoma comprise any one or a combination of A375, SK-mel-2, and M14;

cancer cells of the glioblastoma comprise U87-MG;

cancer cells of the neuroblastoma comprise SH-SY5Y;

cancer cells of the glioma comprise SHG-44;

cancer cells of the osteosarcoma comprise any one or a combination of MG-63, Saos-2, and U-2OS;

cancer cells of the lymphoma comprise any one or a combination of U937, Raji, U2932, WSU-DLCL2, OCI-LY10, and JeKo-1;

cancer cells of the hematologic malignancies comprise any one or a combination of NB4, Reh, K562, Thp-1, JurKat, and HL60;

cancer cells of the myeloma comprise any one or a combination of NCI-H929, ARP-1 and RPMI-8226;

cancer cells of the cholangiocarcinoma comprise QBC939;

and/or,

cancer cells of the prostate cancer comprise Du145 cells.

5. The use of a micropeptide MIAC in preparation of a reagent or a medicament for detecting, preventing or treating tumors according to claim 3, wherein the preventing or treating tumors comprises inhibiting the migration of prostate cancer Du145 cells, cervical cancer Hela cells, osteosarcoma MG-63 cells, ovarian cancer SKOV3 cells, melanoma A375 cells, and/or hepatocellular carcinoma Hep3B cells.

6. The use of a micropeptide MIAC in preparation of a reagent or a medicament for detecting, preventing or treating tumors according to claim 3, wherein the preventing or treating tumors comprises inhibiting the growth of hepatocellular carcinoma Hep3B cells.

7. Use of a nucleotide in preparation of a reagent or a medicament for detecting, preventing or treating tumors, wherein the nucleotide encodes an amino acid sequence as shown in SEQ ID NO: 1; and the tumors comprise one or more of pancreatic cancer, hepatocellular carcinoma, colorectal cancer, ovarian cancer, cervical cancer, bladder cancer, melanoma, glioblastoma, neuroblastoma, glioma, osteosarcoma, lymphoma, hematologic malignancies, myeloma, cholangiocarcinoma and prostate cancer.

8. The use of nucleotide in preparation of a reagent or a medicament for detecting, preventing or treating tumors according to claim 7, wherein the nucleotide comprises a nucleotide sequence as shown in SEQ ID NO: 2.

9. Use of a recombinant vector in preparation of a reagent or a medicament for detecting, preventing or treating tumors, wherein the recombinant vector encodes an amino acid sequence as shown in SEQ ID NO: 1 or comprises a nucleotide sequence as shown in SEQ ID NO: 2; and the tumors comprise one or more of pancreatic cancer, hepatocellular carcinoma, colorectal cancer, ovarian cancer, cervical cancer, bladder cancer, melanoma, glioblastoma, neuroblastoma, glioma, osteosarcoma, lymphoma, hematologic malignancies, myeloma, cholangiocarcinoma and prostate cancer.

10. A pharmaceutical composition for treating tumors, wherein the pharmaceutical composition comprises at least the micropeptide MIAC according to claim 1, the nucleotide according to claim 7 or 8, or the recombinant vector according to claim 9, and a pharmaceutically acceptable carrier thereof; and the tumors comprise one or more of pancreatic cancer, hepatocellular carcinoma, colorectal cancer, ovarian cancer, cervical cancer, bladder cancer, melanoma, glioblastoma, neuroblastoma, glioma, osteosarcoma, lymphoma, hematologic malignancies, myeloma, cholangiocarcinoma and prostate cancer.

**FIG. 1**

**FIG. 2A**

**FIG. 2B**

**Hela**

**FIG. 3A**

0 µM     6.25 µM     12.5 µM

25 µM     50 µM

**FIG. 3B**

**MG-63**

**FIG. 4A**

**0 μM** **3.125 μM** **6.25 μM**

**12.5 μM** **25 μM**

**FIG. 4B**

SKOV3

**FIG. 5A**

**0 μM** **12.5 μM**

**25 μM** **50 μM**

**FIG. 5B**

**FIG. 6A**

**FIG. 6B**

**FIG. 7A**

**0 µM**　　　　**12.5 µM**

**25 µM**　　　　**50 µM**

**FIG. 7B**

Hep3B

- ● Blank group
- ■ Oxaliplatin (15 mg/kg)
- ▲ Sorafenib (50 mg/kg)
- ▼ MIAC 5 mg/kg
- ◆ MIAC 10 mg/kg
- ● MIAC 15 mg/kg
- ■ MIAC 20 mg/kg

**FIG. 8**

Hep3B

**FIG. 9**

## INTERNATIONAL SEARCH REPORT

| International application No. |
|---|
| **PCT/CN2024/093260** |

**A. CLASSIFICATION OF SUBJECT MATTER**

C07K14/47(2006.01)i; C12N15/12(2006.01)i; C12Q1/6886(2018.01)i; C12N15/85(2006.01)i; A61K38/16(2006.01)i; A61P35/00(2006.01)i; G01N33/68(2006.01)i; G01N33/574(2006.01)i

According to International Patent Classification (IPC) or to both national classification and IPC

**B. FIELDS SEARCHED**

Minimum documentation searched (classification system followed by classification symbols)

IPC: C07K; C12N; C12Q; A61K; A61P; G01N

Documentation searched other than minimum documentation to the extent that such documents are included in the fields searched

Electronic data base consulted during the international search (name of data base and, where practicable, search terms used)

CNKI, CNABS, CNTXT, DWPI, EPTXT, WOTXT, USTXT, ELSEVIER, ISI Web of Knowledge, PubMed, Bing, 万方, WANFANG, 百度, BAIDU, NCBI, STN: MIAC, micropeptide inhibiting actin cytoskeleton, 微肽, micropeptide, lncRNA, 长链非编码RNA, long non coding RNA, 肿瘤, tumor, 生长, grow, 迁移, migration, 增殖, proliferation, 胰腺癌, 肝癌, 结直肠癌, 卵巢癌, 宫颈癌, 膀胱癌, 黑色素瘤, 胶质母细胞瘤, 神经母细胞瘤, 胶质瘤, 骨肉瘤, 淋巴瘤, 血液瘤, 骨髓瘤, 胆管癌, 前列腺癌, pancreatic cancer, liver cancer, colorectal cancer, ovarian cancer, cervical cancer, bladder cancer, melanoma, glioblastoma, neuroblastoma, glioma, osteosarcoma, lymphoma, haematoma, myeloma, cholangiocarcinoma, prostate cancer, SEQ ID NOs: 1-2

**C. DOCUMENTS CONSIDERED TO BE RELEVANT**

| Category* | Citation of document, with indication, where appropriate, of the relevant passages | Relevant to claim No. |
|---|---|---|
| X | CN 112442116 A (NANJING ANJI BIOTECHNOLOGY CO., LTD.) 05 March 2021 (2021-03-05) claims 1-10, description, paragraphs 0013-0032, embodiment 8, and table 7 | 1-10 |
| A | CN 110064045 A (SOOCHOW UNIVERSITY) 30 July 2019 (2019-07-30) entire document | 1-10 |
| A | CN 112442537 A (CHINA PHARMACEUTICAL UNIVERSITY) 05 March 2021 (2021-03-05) entire document | 1-10 |
| A | CN 110215511 A (SOOCHOW UNIVERSITY) 10 September 2019 (2019-09-10) entire document | 1-10 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **31 July 2024** | **06 August 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/093260** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2021017242 A1 (FUNDACIO PRIVADA INSTITUTD INVESTIGACIO ONCOLOGICA DE VALL HEBRON) 21 January 2021 (2021-01-21)<br>entire document | 1-10 |
| A | LI, Mengwei et al. "Micropeptide MIAC Inhibits the Tumor Progression by Interacting with AQP2 and Inhibiting EREG/EGFR Signaling in Renal Cell Carcinoma"<br>*Molecular Cancer*, Vol. 21, 19 September 2022 (2022-09-19), article 181, pages 1-15<br>abstract | 1-10 |

Form PCT/ISA/210 (second sheet) (July 2022)

## INTERNATIONAL SEARCH REPORT

| International application No. |
| --- |
| **PCT/CN2024/093260** |

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

        ☐ accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

International application No.

**PCT/CN2024/093260**

| Patent document cited in search report | | | Publication date (day/month/year) | Patent family member(s) | | | Publication date (day/month/year) |
|---|---|---|---|---|---|---|---|
| CN | 112442116 | A | 05 March 2021 | None | | | |
| CN | 110064045 | A | 30 July 2019 | None | | | |
| CN | 112442537 | A | 05 March 2021 | None | | | |
| CN | 110215511 | A | 10 September 2019 | None | | | |
| US | 2021017242 | A1 | 21 January 2021 | WO | 2019175376 | A1 | 19 September 2019 |
| | | | | EP | 3539975 | A1 | 18 September 2019 |
| | | | | JP | 2021520845 | A | 26 August 2021 |
| | | | | US | 11858972 | B2 | 02 January 2024 |
| | | | | EP | 3765483 | A1 | 20 January 2021 |
| | | | | US | 2024182532 | A1 | 06 June 2024 |
| | | | | AU | 2019235440 | A1 | 12 November 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 112442116 A

**Non-patent literature cited in the description**

- **ZHANG M et al.** A peptide encoded by circular form of LINC-PINT suppresses oncogenic transcriptional elongation in glioblastoma. *Nat Commun.*, 26 October 2018, vol. 9 (1), 4475

- **XU SHUYUN**. Experimental Methodology of Pharmacology. People's Medical Publishing House, 1991